# EUROPEAN PATENT APPLICATION

(11) **EP 1 333 033 A1**
(43) Date of publication of application: **06.08.2003**
(21) Application number: 02002222.4
(22) Date of filing: 30.01.2002
(51) Int. Cl.: C07K 5/06, A61K 47/48

(54) **FAP-activated anti-tumor compounds**

(71) Applicant: Boehringer Ingelheim Pharma GmbH & Co.KG, 55216 Ingelheim am Rhein (DE)
(72) Inventor: Peters, Stefan, 88400 Biberach (DE); Breitfelder, Steffen, 88433 Assmannshardt (DE); Park, John Edward, 88400 Biberach (DE); Garin-Chesa, Pilar, 1230 Wien (AT); Blech, Stefan Matthias, 88447 Warthausen (DE); Lenter, Martin, 89073 Ulm (DE)

(57) **Abstract**

The invention relates to a prodrug that is capable of being converted into a drug by the catalytic action of human fibroblast activation protein (FAPα), said prodrug is chemically stable under physiological conditions and can be used for the manufacture of physically stable aqueous formulations. It has a cleavage site which is recognised by FAPα, and the drug released by the enzymatic activity of FAPα is cytotoxic or cytostatic under physiological conditions.

## Description

### TECHNICAL FIELD OF THE INVENTION

The present invention relates to the field of tumor treatment by administration of a prodrug that is converted into a drug at the site of the tumor. In particular, the invention relates to prodrugs which may be converted into a drug by the catalytic action of FAPα, their manufacture and pharmaceutical use.

### BACKGROUND OF THE INVENTION

The human fibroblast activation protein (FAPα) is a Mᵣ 95,000 cell surface molecule originally identified with monoclonal antibody (mAb) F19 (Rettig *et al*. (1988) *Proc. Natl. Acad. Sci. USA* **85**, 3110-3114; Rettig *et al*. (1993) *Cancer Res.* **53,** 3327-3335). The FAPα cDNA codes for a type II integral membrane protein with a large extracellular domain, transmembrane segment, and short cytoplasmic tail (Scanlan *et al.* (1994) *Proc. Natl. Acad. Sci. USA* **91,** 5657-5661; WO 97/34927). FAPα shows 48 % amino acid sequence identity to the T-cell activation antigen CD26, also known as dipeptidyl peptidase IV (DPPIV; EC 3.4.14.5), a membrane-bound protein with dipeptidyl peptidase activity (Scanlan *et al., loc. cit.*). FAPα has enzymatic activity and is a member of the serine protease family, with serine 624 being critical for enzymatic function (WO 97/34927). Work using a membrane overlay assay revealed that FAPα dimers are able to cleave Ala-Pro-7-amino-4-trifluoromethyl coumarin, Gly-Pro-7-amino-4-trifluoromethyl coumarin, and Lys-Pro-7-amino-4-trifluoromethyl coumarin dipeptides (WO 97/34927).

FAPα is selectively expressed in reactive stromal fibroblasts of many histological types of human epithelial cancers, granulation tissue of healing wounds, and malignant cells of certain bone and soft tissue sarcomas. Normal adult tissues are generally devoid of detectable FAPα, but some fetal mesenchymal tissues transiently express the molecule. In contrast, most of the common types of epithelial cancers, including >90% of breast, non-small-cell lung, and colorectal carcinomas, contain FAPα-reactive stromal fibroblasts (Scanlan *et al., loc. cit.*).

These FAPα⁺ fibroblasts accompany newly formed tumor blood vessels, forming a distinct cellular compartment interposed between the tumor capillary endothelium and the basal aspect of malignant epithelial cell clusters (Welt *et al*. (1994) *J*. *Clin. Oncol.* **12**(6), 1193-1203). While FAPα⁺ stromal fibroblasts are found in both primary and metastatic carcinomas, the benign and premalignant epithelial lesions tested (Welt et al., loc. cit.), such as fibroadenomas of the breast and colorectal adenomas, only rarely contain FAPα⁺ stromal cells. Based on the restricted distribution pattern of FAPα in normal tissues and its uniform expression in the supporting stroma of many malignant tumors, clinical trials with ¹³¹I-labeled mAb F19 have been initiated in patients with metastatic colon carcinomas (Welt *et al*., *loc. cit.*).

For new cancer therapies based on cytotoxic or cytostatic drugs, a major consideration is to increase the therapeutic index by improving the efficacy of cancerous tissue killing and/or reducing the toxicity for normal tissue of the cytotoxic or cytostatic agents. To increase specificity of tumor tissue killing and reduce toxicity in normal tissues, trigger mechanisms can be designed so that the toxic agents synthesized in their prodrug or inactive forms are rendered active when and where required, notably in the cancerous tissues (Panchal (1998) *Biochem. Pharmacol.* **55,** 247-252). Triggering mechanisms may include either exogenous factors such as light or chemicals or endogenous cellular factors, such as enzymes with restricted expression in cancer tissues. Another concept, that has been further elaborated, is called 'antibody-directed enzyme prodrug therapy' (ADEPT) or 'antibody-directed catalysis' (ADC) (Huennekens (1994) *Trends Biotechnol*. **12**, 234-239; Bagshawe (1994) *Clin. Pharmacokinet*. **27**, 368-376; Wang *et al.* (1992) *Cancer Res.* **52**, 4484-4491; Sperker *et al.* (1997) *Clin. Pharmacokinet.* **33**(1), 18-31). In ADEPT, an antibody directed at a tumor-associated antigen is used to target a specific enzyme to the tumor site. The tumor-located enzyme converts a subsequently administered prodrug into an active cytotoxic agent. The antibody-enzyme conjugate (AEC) binds to a target antigen on cell membranes or to free antigen in extracellular fluid (ECF). A time interval between giving the AEC and prodrug allows for the AEC to be cleared from normal tissues so that the prodrug is not activated in the normal tissues or blood. However, some disadvantages of ADEPT are related to the properties of the AEC (Bagshawe, *loc. cit.*). For example, in humans, only a small fraction of the administered dose of the targeting AEC binds to tumor tissue and the remainder is distributed through body fluids from which it is cleared with significant time delays. Even very low concentrations of unbound enzyme can catalyze enough prodrug to have toxic effects because plasma and normal ECF volumes are much greater than those of tumor ECF. The AEC may also be immunogenic, thus preventing repeat administration, in many instances.

The International patent applications WO 97/12624 and WO 97/14416 disclose oligopeptides including the following penta- and hexapeptide (SEQ.ID.NOs.: 151 and 177: hArg-Tyr-Gln-Ser-Ser-Pro; hArg-Tyr-Gln-Ser-Pro;), comprising amino acid sequences, which are recognized and proteolytically cleaved by free prostate specific antigen (PSA) and therapeutic agents which comprise conjugates of such oligopeptides and known therapeutic or cytotoxic agents. These oligopeptide conjugates which comprise at least one glutamine-serine moiety are useful for treatment of prostate cancer only.

The International patent application WO 00/71571 discloses prodrugs which are capable of being converted into a drug by the catalytic action of FAPα, said prodrug having a cleavage site which is recognized by FAPα, and said drug being cytotoxic or cytostatic under physiological conditions. However, these prodrugs have comparably poor solubilities.

The problem underlying the present invention was to provide similar prodrugs with improved solubilities.

### BRIEF DESCRIPTION OF THE INVENTION

Surprisingly it has been found that the prodrugs, wherein the N-terminal amino function of the oligomeric part is attached to a capping group (Cg) of formula (II) 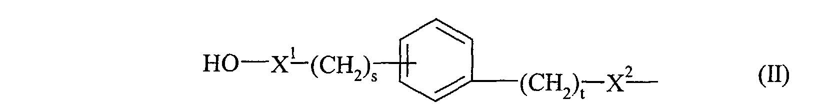 in which
X¹ represents C=O or SO₂,
X² represents C=O, SO₂, NH-C=O or a single bond,
s is an integer of 1 or 2, and
t is 0 or an integer of 1, 2 or 3
and have high solubilities.

The present invention relates to prodrugs which are capable of being converted into a cytotoxic or cytostatic drug, by the catalytic action of FAPα, said prodrugs exhibit an oligomeric part comprising up to 13 amino carboxylic acid residues, the C-terminal amino carboxylic acid thereof is recognized by FAPα, and a cytotoxic or cytostatic part, wherein the N-terminal amino function of the oligomeric part is attached to a capping group (Cg) of formula II.

In the context of this invention, a "drug" shall mean a chemical compound that may be administered to humans or animals as an aid in the treatment of disease. In particular, a drug is an active pharmacological agent.

The term "cytotoxic compound" shall mean a chemical compound which is toxic to living cells, in particular a drug that destroys or kills cells. The term "cytostatic compound" shall mean a compound that suppresses cell growth and multiplication and thus inhibits the proliferation of cells. Examples for cytotoxic or cytostatic compounds suitable for the present invention are anthracycline derivatives such as doxorubicin, analogs of methotrexate such as methothrexate, pritrexime, trimetrexate or DDMP, melphalan, analogs of cisplatin such as cisplatin, JM216, JM335, bis(platinum) or carboplatin, analogs of purines and pyrimidines such as cytarbine, gemcitabine, azacitidine, 6-thioguanine, flurdarabine or 2-deoxycoformycin, and analogs of other chemotherapeutic agents such as 9-aminocamptothecin, D,L-aminoglutethimide, trimethoprim, pyrimethamine, mitomycin C, mitoxantrone, cyclophosphanamide, 5-fluorouracil, extramustine, podophyllotoxin, bleomycin, epothilone and derivatives of epothilone as described for example in US 6,204,388 or taxol.

A "prodrug" shall mean a compound that, on administration, must undergo chemical conversion by metabolic processes before becoming an active pharmacological agent. In particular, a prodrug is a precursor of a drug. In the context of the present invention, the prodrug is significantly less cytotoxic or cytostatic than the drug it is converted into upon the catalytic action of FAPα. The expert knows methods of determining cytotoxicity of a compound, see e.g. example 6 of WO 00/71571, or Mosmann ((1983) *J*. *Immun. Meth*. **65**, 55-63). Preferably, the prodrug is at least three times less cytotoxic as compared to the drug in an in vitro assay.

A "drug being cytostatic or cytotoxic under physiological conditions" shall mean a chemical compound which is cytostatic or cytotoxic in a living human or animal body, in particular a compound that kills cells or inhibits proliferation of cells within a living human or animal body.

A "prodrug having a cleavage site which is recognised by FAPα" shall mean a prodrug which can act as a substrate for the enzymatic activity of FAPα. In particular, the enzymatic activity of FAPα can catalyse cleavage of a covalent bond of the prodrug under physiological conditions. By cleavage of this covalent bond, the prodrug is converted into the drug, either directly or indirectly. Indirect activation would be the case if the cleavage product of the FAPα catalyzed step is not the pharmacologically active agent itself but undergoes a further reaction step, e.g. hydrolysis, to become active. More preferably, the cleavage site of the prodrug is specifically recognized by FAPα, but not by other proteolytic enzymes present in the human or animal body. Also preferably, the cleavage site is specifically recognised by FAPα, but not by proteolytic enzymes present in human or animal body fluids, especially plasma. In a particularly preferred embodiment, the prodrug is stable in plasma, other body fluids, or tissues, in which biologically active FAPα is not present or detectable. Preferably, in an *in vitro* assay as carried out in Example 7 of the International Patent Application WO 00/71571, the disclosure of which is fully incorporated herein by reference, more than 50%, more preferably more than 80%, more preferably more than 90% of the prodrug are still present in a solution containing 10% (v/v) of human plasma after 8 h at 37°C. The cleavage site should most preferably be specific for FAPα. In a preferred embodiment, the cleavage site comprises a L-proline residue which is linked to a cytotoxic or cytostatic drug via an amide bond. An example of this class is a doxorubicin-peptide conjugate. FAPα may catalyse the cleavage of a peptidic bond between the C-terminal amino acid residue of the peptide, which is preferably L-proline, and the cytotoxic or cytostatic compound.

Preferred compounds show at least 10% conversion to free drug, under standard conditions listed below. More preferred are compounds that show at least 20% conversion to free drug, under standard conditions. Even more preferred are compounds that show at least 50% conversion to free drug, under standard conditions. In this context, standard conditions are defined as follows: Each compound is dissolved in 50 mM Hepes buffer, 150 mM NaCl, pH 7.2, at a final concentration of 5 µM and incubated with 100 ng CD8FAPα (see example 4 of WO 00/71571) for 24 hours at 37 °C. Release of free drug by CD8FAPα is determined as described in example 5 of WO 00/71571.

### DETAILED DESCRIPTION OF THE INVENTION

Preferably, the present invention relates to a compound of formula (I) 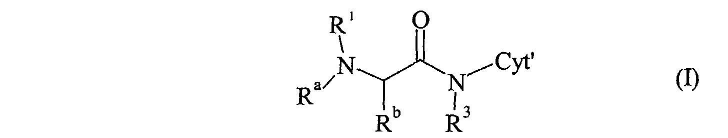 or a pharmaceutically acceptable salt thereof,
wherein
R¹ represents an amino alkanoyl or oligopeptidoyl group, the N-terminal amino function of which is attached to a capping group (Cg) of formula (II) 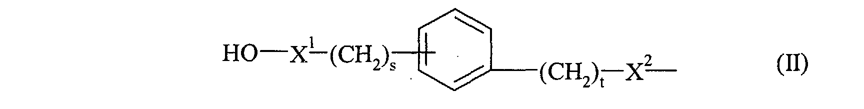 in which
X¹ represents C=O or SO₂,
X² represents C=O, SO₂, NH-C=O or a single bond,
s is an integer of 1 or 2, and
t is 0 or an integer of 1, 2 or 3,
R^{a} and R^{b} together with the interjacent N-C group form an optionally substituted, optionally benzo- or cyclohexano-condensed 3- to 7-membered saturated or unsaturated heterocyclic ring, in which one or two CH₂ groups may also be replaced by NH, O or S; R³ represents H, C₁-C₆-alkyl, C₃-C₈-cycloalkyl, aryl or heteroaryl; and
Cyt' represents the residue of a cytotoxic or cytostatic compound.

Preferred are the compounds of formula I, wherein
X¹ represents C=O or SO₂, in particular C=O,
X² represents C=O or SO₂, in particular C=O,
s is an integer of 1 or 2, in particular 1,and
t is an integer of 1 or 2, in particular 1.

Particularly preferred are the capping groups, wherein the group HO-X¹-(CH₂)ₛ- is attached in the the *meta-* or *para*-position with respect to the group -(CH₂)ₜ-X²-. Most preferred are the capping groups selected from the formulae IIA and IIB:

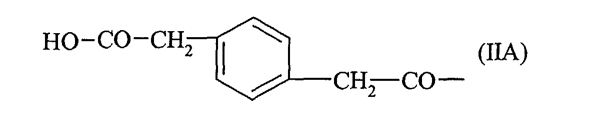

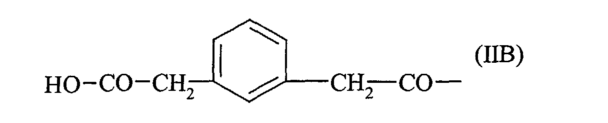

Furthermore preferred are those compounds of formula I, wherein
R¹ represents a residue of formula Cg-A, Cg-B-A or Cg-(D)ₙ-B-A, in which
Cg represents a capping group of formula (II),
A, B and D each independently represent moieties derived from amino carboxylic acids of the formula -[NR⁴-(X)ₚ-CO]- wherein X represents CR⁵R⁶ and wherein R⁴, R⁵ and R⁶ each independently represent a hydrogen atom, an optionally substituted C₁-C₆-alkyl, C₃-C₈-cycloalkyl, aryl, aralkyl, heteroaryl or heteroarylalkyl group, and p is 1, 2, 3, 4, 5; or
A, B and D each independently represent moieties derived from cyclic amino carboxylic acids of formula 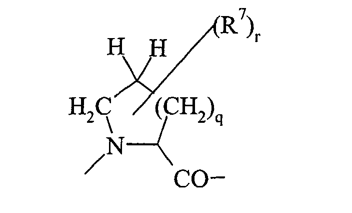 wherein
R⁷ represents C₁-C₆-alkyl, OH, or NH₂,
n is an integer from 1 to 10;
q is 0, 1 or 2; and
r is 0, 1 or 2, in particular wherein
R¹ represents a group selected from Cg-A, Cg-B-A- and Cg-(D)ₙ-B-A-in which A, B and D are amino acid moieties, which are each independently selected from glycine (Gly), and the D- or L-forms of alanine (Ala), valine (Val), leucine (Leu), isoleucine (Ile), phenylalanine (Phe), tyrosine (Tyr), tryptophan (Trp), cysteine (Cys), methionine (Met), serine (Ser), threonine (Thr), lysine (Lys), arginine (Arg), histidine (His), aspartatic acid (Asp), glutamic acid (Glu), asparagine (Asn), glutamine (Gln), proline (Pro), 4-hydroxy-proline (Hyp), 5-hydroxy-lysine, norleucine (Nle), 5-hydroxynorleucine (Hyn), 6-hydroxynorleucine, ornithine, cyclohexylglycine (Chg), N-methylglycin (N-MeGly), N-methylalanin (N-MeAla), N-methylvaline (N-MeVal), N-methylleucine (N-MeLeu), N-methylisoleucine (N-MeIle), N-methylnorleucin (N-MeNle), N-methyl-2-aminobutyric acid (N-MeAbu) and N-methyl-2-aminopentanoic acid (N-MeNva).

The unit A is preferably selected from L-proline, glycine, L-norleucine, L-cyclohexylglycine, L-5-hydroxynorleucine, L-6-hydroxynorleucine, L-5-hydroxylysine, L-arginine, and L-lysine.

The N-terminal unit D is preferably an amino acid, wherein the amino group thereof is a secondary amino group forming with the linkage to the capping group a tertiary amino group, in particular selected from L-proline (Pro), azetidine-2-ylcarboxylic acid and N-C₁₋₆ alkyl amino acids such as N-methylglycin (N-MeGly), N-methylalanin (N-MeAla), N-methylvaline (N-MeVal), N-methylleucine (N-Me-Leu), N-methylisoleucine (N-Me-Ile), N-methylnorleucin (N-MeMle), N-methyl-2-aminobutyric acid (N-Me-Abu) and N-methyl-2-aminopentanoic acid (N-MeNva).

Furthermore preferred are those compounds of formula I, wherein the heterocyclic ring formed by R^{a}, R^{b} and the interjacent N-C is substituted by R⁸ and R⁹, wherein R⁸ and R⁹ each independently represent a hydrogen or halogen atom or a C₁-C₆-alkyl, C₁-C₆-alkylamino, di-C₁-C₆-alkylamino, C₁-C₆-alkoxy, thiol, C₁-C₆-alkylthio, oxo, imino, fomyl, C₁-C₆-alkoxy carbonyl, amino carbonyl, C₃-C₈-cycloalkyl, aryl, or heteroaryl group.

Particularly preferred are the compounds of formula IA, 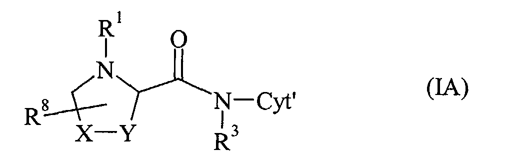 wherein R¹, R³, R⁸, Cyt' are as hereinbefore, and
X-Y represents CHR⁹-CH₂, CR²=CH, NH-CH₂, CH₂-NH, -CR⁹-, CH₂-CHR⁹-CH₂, in particular CH₂-CH₂.

Most preferred are the compounds of formulae IA1 and IA2, 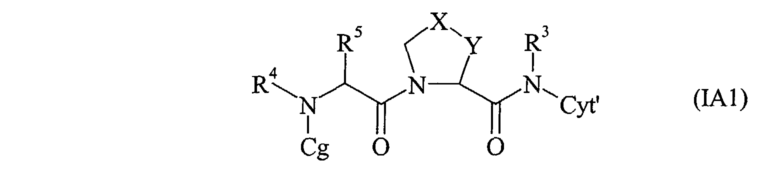 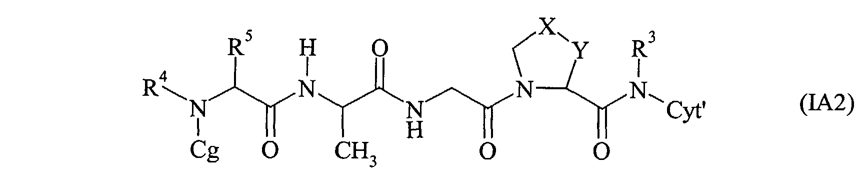 wherein R³, R⁴, R₅, Cyt', Cg, X and Y are as defined hereinbefore, or
R⁴ and R⁵ together with the interjacent N-C group form an optionally substituted, optionally benzo- or cyclohexano-condensed 3- to 7-membered saturated or unsaturated heterocyclic ring, in which one or two CH₂ groups may also be replaced by NH, O or S.

Unless indicated otherwise, the simple stereoisomers as well as mixtures or racemates of the stereoisomers are included in the invention.

"C₁-C₆-alkyl" generally represents a straight-chained or branched hydrocarbon radical having 1 to 6 carbon atoms.

The term "optionally substituted" as used hereinabove or hereinbelow with respect to a group or a moiety refers to a group or moiety which may optionally be substituted by one or several halogen atoms, hydroxyl, amino, C₁-C₆-alkyl-amino, di- C₁-C₆-alkyl-amino, C₁-C₆-alkyl-oxy, thiol, C₁-C₆-alkyl-thio, =O, =NH, -CHO, -COOH, -CONH₂, -NHC(=NH)NH₂, , C₃-C₈-cycloalkyl, aryl, or heteroaryl substituents , which may be identical to one another or different.

### The following radicals may be mentioned by way of example:

Methyl, ethyl, propyl, 1-methylethyl (isopropyl), butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2methylpropyl, HOCH₂-, CH₃CH(OH)-, CH₃CH(OH)CH₂CH₂-, HOCH₂CH₂CH₂CH₂-,
H₂NCH₂CH₂CH₂-, H₂NCH₂CH₂CH₂CH₂-, H₂NCH₂CH(OH)CH₂CH₂-, H₂NC(=NH)NHCH₂CH₂CH₂-, HSCH₂-, CH₃SCH₂CH₂-, HOOCCH₂-, HOOCCH₂CH₂-, H₂NC(=O)CH₂-, H₂NC(=O)CH₂CH₂-, benzyl, *para*-hydroxy-benzyl, 4-(*para*-hydroxyphenoxy)-benzyl, 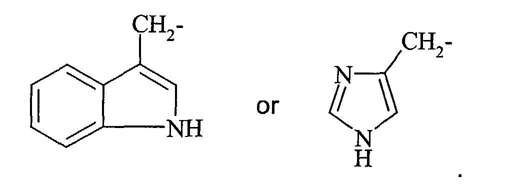 If a C₁-C₆-alkyl group is substituted, the substituents are preferably hydroxyl, amino, dimethylamino, diethylamino, thiol, methyl-thiol, methoxy, ethoxy, =O, =NH, -CHO, -COOH, -COOCH₃, -COOCH₂CH₃, -CONH₂, -NHC(=NH)NH₂, cyclohexyl, phenyl, benzyl, para-hydroxy-benzyl, 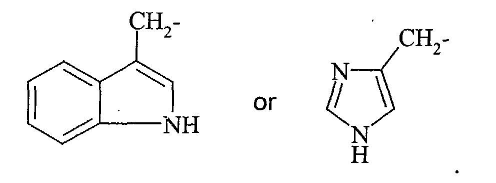 If C₁-C₆-alkyl is substituted with aryl or heteroaryl, C₁-C₆-alkyl is preferably C₁, more preferably a methylene group.

The terms "amino alkanoyl" and "oligopeptidoyl" including "di- or tripeptidoyl" as used hereinabove or hereinbelow with respect to radical R¹ describe a radical in which an amino acid or an oligomer comprising up to 12, preferably 2 or 3 amino acid moieties is attached C-terminally to the nitrogen atom of the heterocyclic ring via an amide bond.

A person of ordinary skill in the chemistry of amino acids and oligopeptides will readily appreciate that certain amino acids may be replaced by other homologous, isosteric and/or isolectronic amino acids wherein the biological activity of the original amino acid or oligopeptide has been conserved upon modification. Certain unnatural and modified natural amino acids may also be utilized to replace the corresponding natural amino acid. Thus, for example, tyrosine may be replaced by 3-iodotyrosine, 2- or 3-methyltyrosine, 3-fluorotyrosine.

"C₃-C₈-Cycloalkyl" generally represents cyclic hydrocarbon radical having 3 to 8 carbon atoms which may optionally be substituted by one or several hydroxyl, amino, C₁-C₆-alkyl-amino, di- C₁-C₆-alkyl-amino, C₁-C₆-alkyl, C₁-C₆-alkyloxy, thiol, C₁-C₆-alkyl-thio, =O, =NH, -CHO, -COOH, -COOCH₃, -COOCH₂CH₃, -CONH₂, -NHC(=NH)NH₂, or halogen substituents , which may be identical to one another or different.

"Heterocyclic ring" as used hereinabove and hereinbelow with respect to the group formed by R^{a} and R^{b} together with the interjacent N-C group generally represents a 3 to 7-membered, preferably 4-, 5- or 6-membered non-aromatic heterocyclic ring system, containing one nitrogen atom and optionally 1 or 2 additional heteroatoms selected from the group of nitrogen, oxygen and sulfur, which may be substituted by one or several halogen atoms or C₁-C₆-alkyl, C₁-C₆-alkylamino, di-C₁-C₆-alkylamino, C₁-C₆-alkoxy, thiol, C₁-C₆-alkylthio, oxo, imino, fomyl, C₁-C₆-alkoxy carbonyl, amino carbonyl, C₃-C₈-cycloalkyl, aryl, or heteroaryl groups, which may be identical to one another or different, and which optionally may be benzo- or cyclohexano-condensed. Such heterocyclic rings are preferably azetidine or are derived from a fully or partially hydrogenated pyrrole, pyridine, thiazole, isoxazole, pyrazole, imidazole, indole, benzimidazole, indazole, pyridazine, pyrimidine, pyrazin group. Most preferred are azetidine, pyrrolidine, 3,4-dehydropyrrolidine, piperidine, hexahydro-1H-azepine, octahydroindole, imidazolidine, thiazolidine.

If such heterocyclic ring is substituted, the substituents are preferably methyl, ethyl, propyl, 1-methylethyl (isopropyl), butyl, 1-methylpropyl, 2-methylpropyl, 1,1-dimethylethyl, hydroxyl, amino, dimethyl-amino, diethyl-amino, thiol, methyl-thiol, methoxy, ethoxy, -CHO, -COOH, -COOCH₃, -COOCH₂CH₃, or -CONH₂.

"Aryl" generally represents an aromatic ring system with 6 to 10, preferably 6 carbon atoms which may optionally be substituted by one or several hydroxyl, amino, C₁-C₆-alkyl-amino, di- C₁-C₆-alkyl-amino, C₁-C₆-alkyl, C₁-C₆-alkyloxy, thiol, C₁-C₆-alkyl-thio, -CHO, -COOH, -COOCH₃, -COOCH₂CH₃, -CONH₂, or halogen substituents, which may be identical to one another or different, and which optionally may be benzocondensed. Aryl substituents may be preferably derived from benzene, preferred examples being phenyl, 2-hydroxy-phenyl, 3-hydroxy-phenyl, 4-hydroxy-phenyl, 4-amino-phenyl, 2-amino-phenyl, 3-amino-phenyl.

If aryl is substituted, the substituents are preferably methyl, ethyl, propyl, 1-methylethyl (isopropyl), butyl, 1-methylpropyl, 2-methylpropyl, 1,1-dimethylethyl, hydroxyl, amino, dimethyl-amino, diethyl-amino, thiol, methyl-thiol, methoxy, ethoxy, -CHO, -COOH, -COOCH₃, -COOCH₂CH₃, or -CONH₂.

"Heteroaryl" generally represents a 5 to 10-membered aromatic heterocyclic ring system, containing 1 to 5 heteroatoms selected from the group of nitrogen, oxygen, or sulfur, which may optionally be substituted by one or several hydroxyl, amino, C₁-C₆-alkyl-amino, di- C₁-C₆-alkyl-amino, C₁-C₆-alkyl, C₁-C₆-alkyloxy, thiol, C₁-C₆-alkyl-thio, -CHO, -COOH, -COOCH₃, -COOCH₂CH₃, -CONH₂, or halogen substituents, which may be identical to one another or different, and which optionally may be benzocondensed. Heteroaryl substituents may preferably be derived from furane, pyrrole, thiophene, pyridine, thiazole, isoxazole, pyrazole, imidazole, benzofuran, thianaphthene, indole, benzimidazole, indazole, quinoline, pyridazine, pyrimidine, pyrazine, chinazoline, pyrane, purine, adenine, guanine, thymine, cytosine, uracil.

If heteroaryl is substituted, the substituents are preferably methyl, ethyl, propyl, 1-methylethyl (isopropyl), butyl, 1-methylpropyl, 2-methylpropyl, 1,1-dimethylethyl, hydroxyl, amino, dimethyl-amino, diethyl-amino, thiol, methyl-thiol, methoxy, ethoxy, -CHO, -COOH, -COOCH₃, -COOCH₂CH₃, or -CONH₂.

"Residue of a cytotoxic or cytostatic compound" means that the compound H₂N-Cyt', which is released upon cleavage of the amide bond shown in formula (I), is either cytotoxic or cytostatic itself, or may be converted into a cytotoxic or cytostatic compound in a subsequent step.

In the latter case, -Cyt' may be a residue of formula -L-Cyt", wherein L is a linker residue derived from a bifunctional molecule, for instance a diamine H₂N-L'-NH₂, an amino alcohol H₂N-L'-OH, for example p-amino-benzyl alcohol (PABOH), an amino carbonate, for example 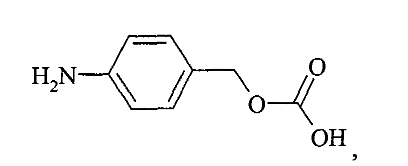 or an unnatural amino carboxylic acid. If -Cyt' is of formula -L-Cyt", the compound H₂N-L'-Cyt" is generated by the enzymatic cleavage of the amide bond shown in formula (I). The compound H₂N-L'-Cyt" may be cytotoxic or cytostatic itself or the linker residue cleaved off from Cyt" in a subsequent step releasing the cytotoxic or cytostatic agent. For example, the compound H₂N-L'-Cyt" may be hydrolysed under physiological conditions into a compound H₂N-L'-OH and the cytotoxic or cytostatic compound H-Cyt", which is the active therapeutic agent (In the following, only the term Cyt' is used for both Cyt' and Cyt", and only the term L is used for both L and L', for simplicity).

The pharmaceutically acceptable salts of the compounds of the present invention include the conventional non-toxic salts formed from non-toxic inorganic or organic acids. For example, such conventional non-toxic salts include those from inorganic acids such as hydrochloric acid, hydrobromic, sulfuric, sulfamic, phosphoric, nitric and the like; and the salts prepared from organic acids such as acetic, propionic, succinic, glycolic, stearic, lactic, malic, tartaric, citric, ascorbic, maleic, phenylacetic, glutamic, benzoic, salicylic, sulfanilic, oxalictrifluoroacetic and the like.

H₂N-Cyt' is preferably an anthracycline derivative of formula IV, 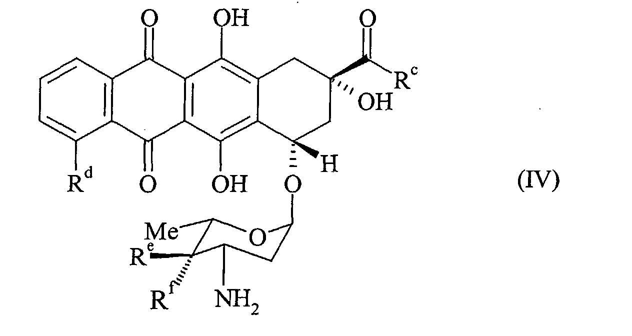 wherein
R^{c} represents C₁-C₆ alkyl, C₁-C₆ hydroxyalkyl or C₁-C₆ alkanoyloxy C₁-C₆ alkyl, in particular methyl, hydroxymethyl, diethoxyacetoxymethyl or butyryloxymethyl;
R^{d} represents hydrogen, hydroxy or C₁-C₆ alkoxy, in particular methoxy;
one of R^{e} and R^{f} represents a hydrogen atom; and the other represents a hydrogen atom or a hydroxy or tetrahydropyran-2-yloxy (OTHP) group.

Paricularly preferred are the following compounds of formula II:

| R^{c} | R^{d} | R^{e} | R^{f} | Cyt |
|---|---|---|---|---|
| CH₂OH | OCH₃ | H | OH | doxorubicin |
| CH₃ | OCH₃ | H | OH | daunorubicin |
| CH₂OH | OCH₃ | OH | H | epirubicin |
| CH₃ | H | H | OH | idarubicin |
| CH₂OH | OCH₃ | H | OTHP | THP |
| CH₂OH | OCH₃ | H | H | esorubicin |
| CH₂OCOCH(OC₂H₅)₂ | OCH₃ | H | OH | detorubicin |
| CH₂OH | H | H | OH | carminorubicin |
| CH₂OCOC₄H₉ | OCH₃ | H | OH | |

Most preferred is doxorubicin (Dox). Other cytotoxic or cytostatic residues Cyt' may be derived for example from methotrexate, trimetrexate, pyritrexim, 5,10-dideazatetrahydrofolatepyrimetamine, trimethoprim, 10-propargyl-5,8-dideazafolate2,4-diamino-5(3',4'-dichloropheyl)-6-methylpyrimidine, aminoglutethimide, goreserelin, melphalan, chlorambucil, analogs of other chemotherapeutic agents such as 9-aminocamptothecin (for examples see e.g. Burris HA, r. d. and S. M. Fields (1994). "Topoisomerase I inhibitors. An overview of the camptothecin analogs. [Review]." *Hematol. Oncol. Clin. North Am*. **8**(2): 333-355; Iyer, L. and M. J. Ratain (1998). "Clinical pharmacology of camptothecins. [Review] [137 refs]." *Cancer Chemother. Pharmacol.* **42** Suppl: S31-S43.) and epothilone and the derivatives thereof.

In formula (I), Cyt' may also be a biological effector molecule which either directly or indirectly effects destruction of tumor cells, like for example TNFα.

Preferred anthracycline prodrugs are the compounds of formula III 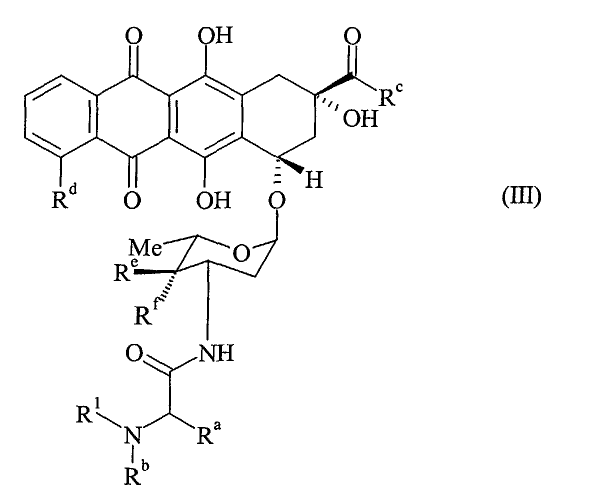 wherein R^{a}, R^{b}, R^{c}, R^{d}, R^{e}, R^{f} and R¹ are as defined hereinabove.

Most preferred compounds of the invention are doxorubicin derivatives of formulae (IIIA) to (IIIK):

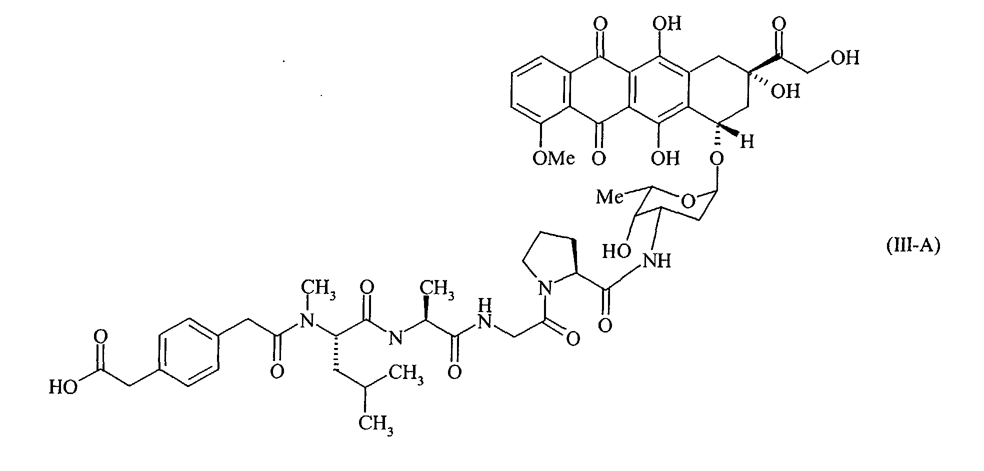

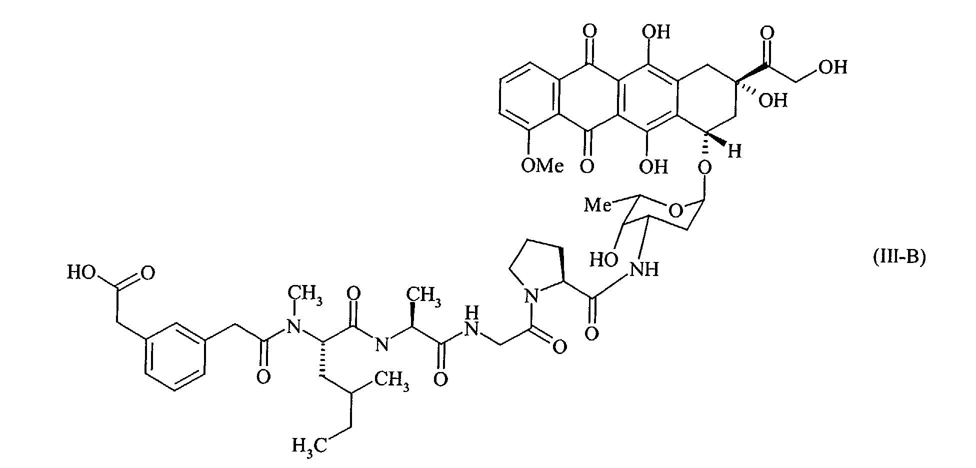

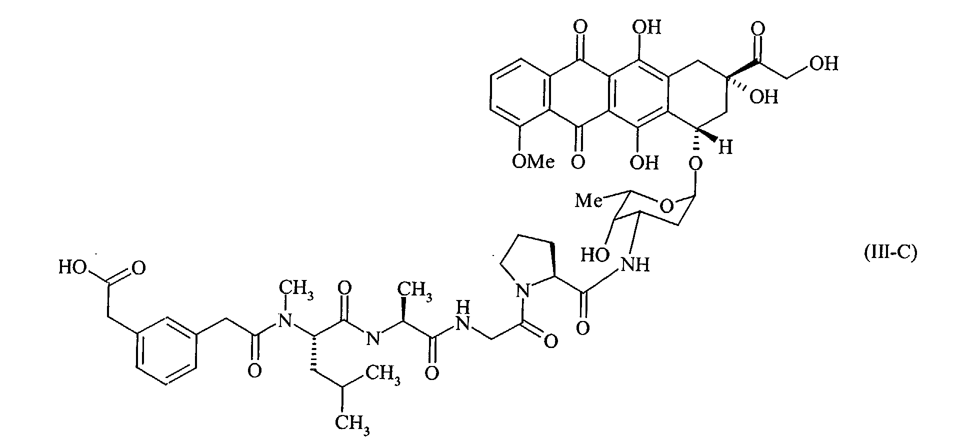

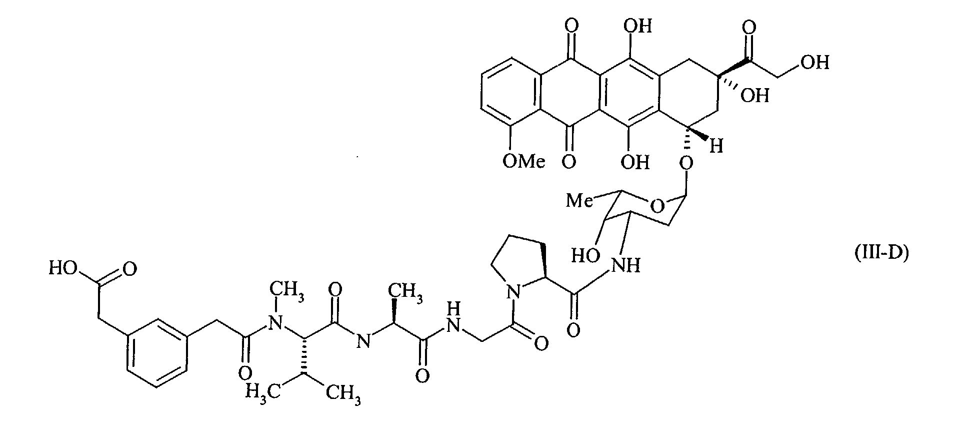

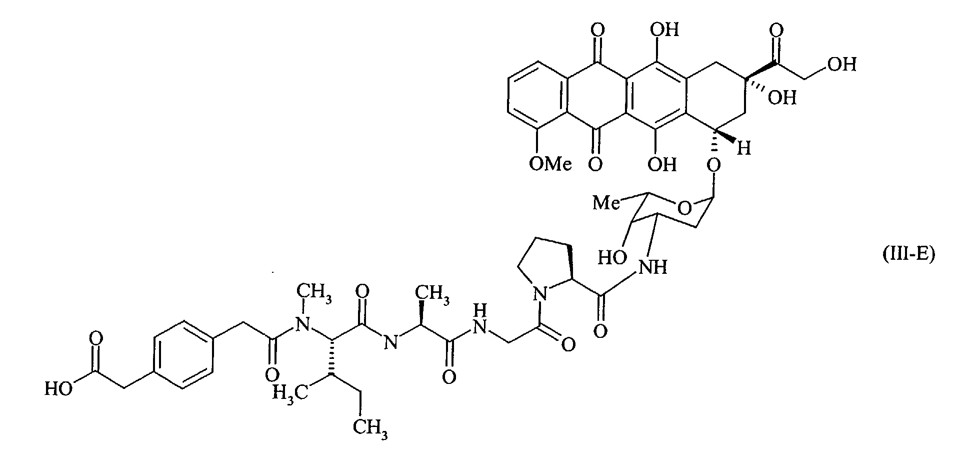

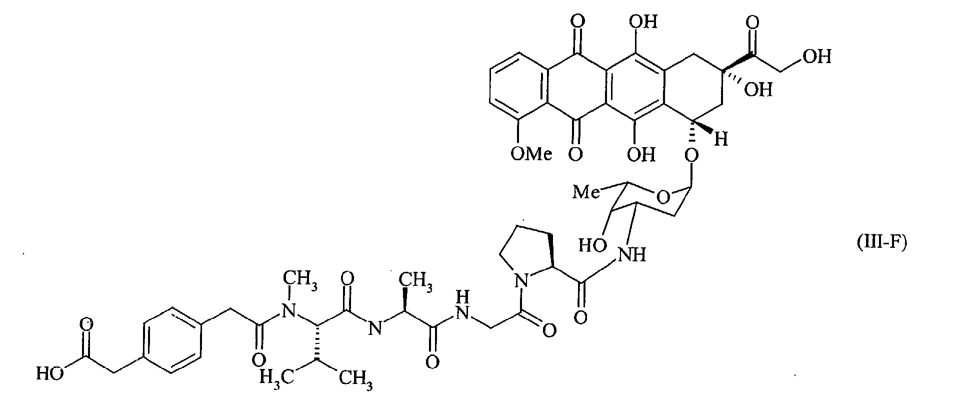

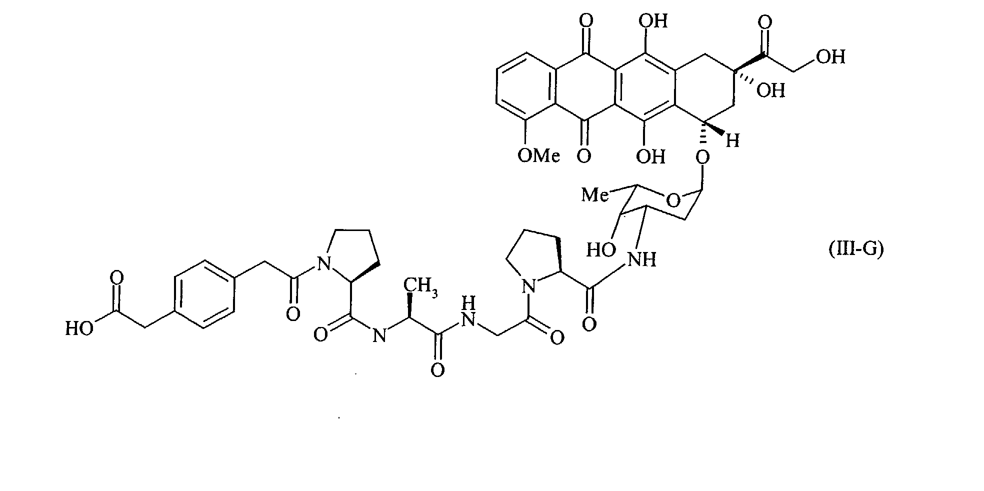

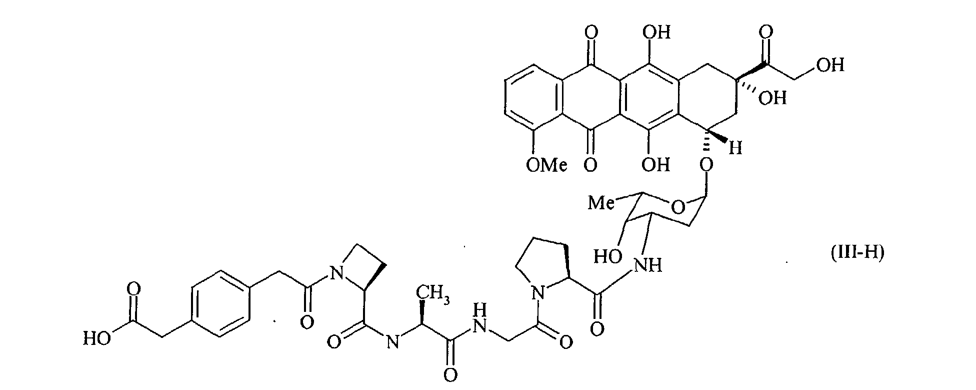

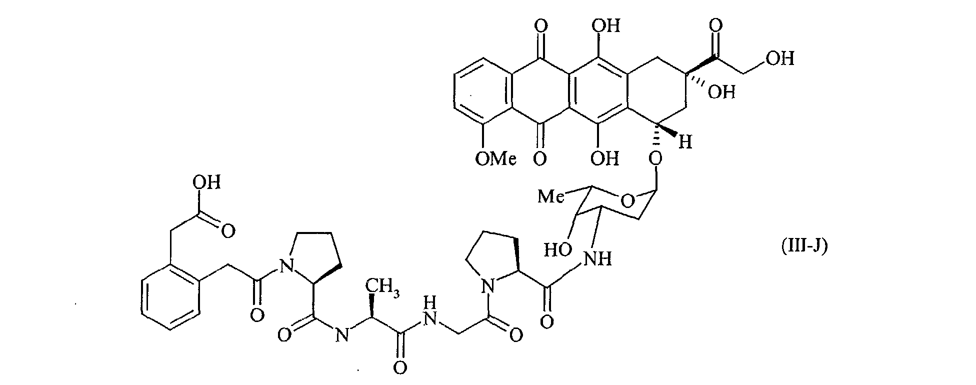

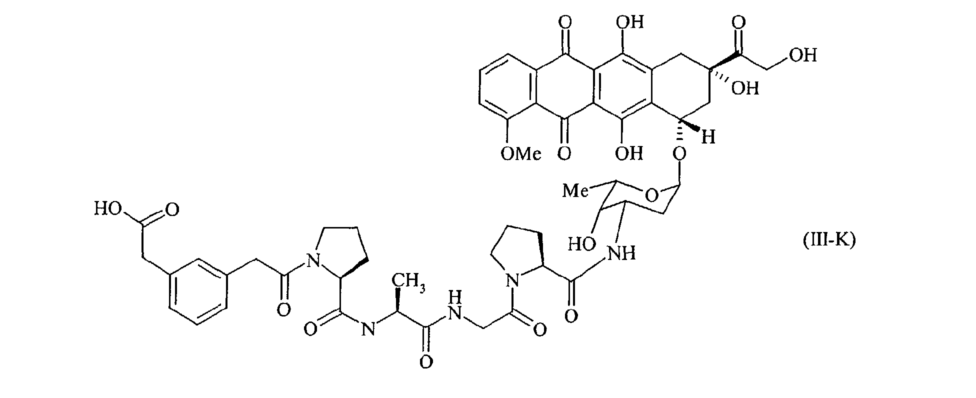

If the part Cg-B-A or Cg-(D)ₘ-B-A of formula (I) contains two or more sulfur atoms, the compound of the invention may contain one or more disulfide bonds.

One class of cytotoxic or cytostatic compounds which may be used for the present invention has a primary amino function which is available for formation of an amidic bond as shown in formula (I), like doxorubicin. In this case, a linker molecule L is not necessary. If a cytostatic or cytotoxic compound does not have such an amino function, such a function may be created in such a compound by way of chemical modification, e.g. by introducing or converting a functional group or attaching a linker molecule to the compound. A linker molecule may also be inserted between the oligomeric part (i.e. the part comprising the amino carboxylic residues) and the cytostatic or cytotoxic part of the compound of the invention to ensure or optimize cleavage of the amide bond between the oligomeric part and the cytotoxic or cytostatic part. If a linker molecule is present, i.e. in compounds containing the structure L-Cyt', the bond between L and Cyt' is preferably an amidic or ester bond. In a preferred embodiment, such a linker molecule is hydrolyzed off the cytostatic or cytotoxic compound under physiological conditions after the enzymatic cleavage and thus the free cytostatic or cytotoxic compound is generated. In any case, the compound of the invention must have the property of being cleavable upon the catalytic action of FAPα and, as a direct or indirect consequence of this cleavage, releasing under physiological conditions a cytostatic or cytotoxic compound.

In a further aspect, the present invention relates to prodrug that is capable of being converted into a cytotoxic or cytostatic drug, by the catalytic action of FAPα, said prodrug exhibits an oligomeric part comprising up to 13 amino carboxylic residues, the C-terminal amino carboxylic thereof is recognized by FAPα, and a cytotoxic or cytostatic part, characterized in that the N-terminal amino function of the oligomeric part is attached to a capping group (Cg) of formula II.

The oligomeric part is preferably a peptide. Preferably, the oligomeric part comprises two, three, four, five, six, seven, eight, nine, ten, eleven, or twelve amino carboxylic acid residues, more preferably two, three, or four amino carboxylic residues.

The compounds of the invention may be synthesized by processes known in the art (E. Wünsch, Synthese von Peptiden, in "Methoden der organischen Chemie", Houben-Weyl (Eds. E. Müller, O. Bayer), Vol. XV, Part 1 and 2, Georg Thieme Verlag, Stuttgart, 1974). For example, the compounds could be synthesized by condensation of the terminal amino function of the oligomeric part of a compound of formula VI with a compound of formula V, 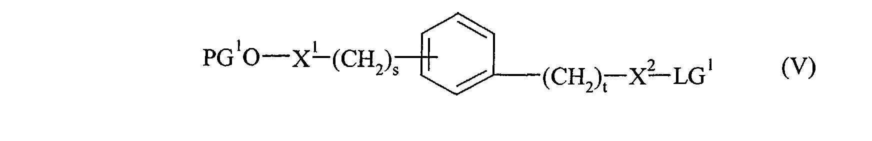 wherein X¹, X², s and t have the meaning given hereinabove, and
LG¹ is OH or an activation leaving group, and
PG¹ is H or a suitable protection group
according to the following reaction scheme: 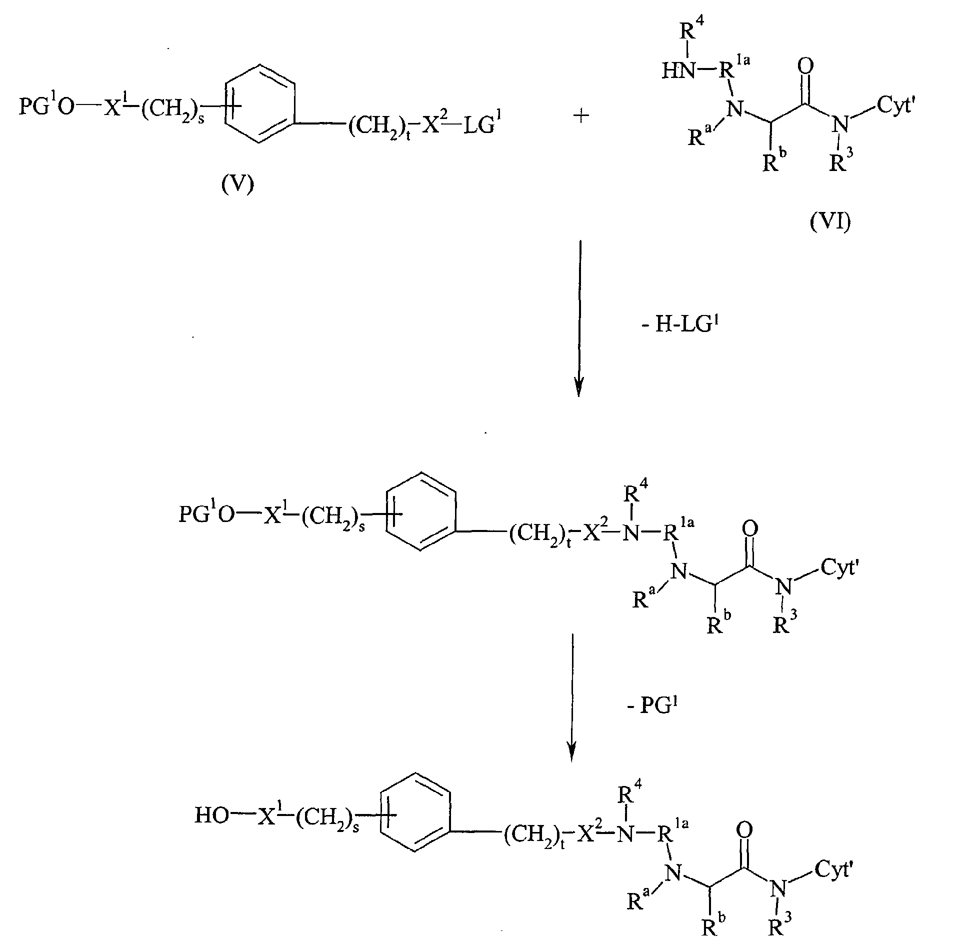 wherein Cyt', R^{a}, R^{b}, R³ and R⁴ have the meaning given hereinabove, and R^{1a} represents an amino alkanoyl or oligopeptidoyl group, the N-terminal amino function of which bears at least one hydrogen atom.

To achieve such an amide formation, it may be necessary to activate the carbonyl group of the carboxylic acid for a nucleophilic attack of an amine, i.e. LG¹ to be an activation group or leaving group which is suitable to be substituted by an amino group. This activation can be done by conversion of the carboxylic acid into an acid chloride or acid fluoride or by conversion of the carboxylic acid into an activated ester, for instance a N-hydroxysuccinimidyl ester or a pentafluorophenyl ester. Another method of activation is the transformation into a symmetrical or unsymmetrical anhydride. Alternatively, the formation of the amide bonds can be achieved by the use of in situ coupling reagents like benzotriazole-1-yl-oxy-tris-pyrrolidino-phosphonium hexafluorophosphate (PyBOP) (E. Frerot et al., *Tetrahedron,* 1991, **47**, 259-70), 1,1'-carbonyldimidazole (CDI) (K. Akaji et al., *THL*, **35**, 1994, 3315-18), 2-(1H-benzotriazole-1-yl)-1,1,3,3-tetramethyluronium tetrafluoroborate (TBTU) (R. Knorr et al., *THL,* **30**, 1989, 1927-30), 1-(mesitylene-2-sulfonyl)-3-nitro-1H-1,2,4-triazole (MSNT) (B. Blankenmeyer-Menge et al., *THL,* **31**, 1990, 1701-04).

The protection group PG¹ is removed at the end of the synthesis.

The compounds of formula VI can be prepared as described in WO 00/71571, the complete disclosure of which is hereby incorporated by reference.

The compounds of the invention are intended for medical use. In particular, these compounds are useful for the treatment of tumors which are associated with stromal fibroblasts that express FAPα and which are generally not optimally treated with available cytotoxic and/or cytostatic agents. Tumors with this property are, for example, epithelial cancers, such as lung, breast, and colon carcinomas. Tumors, such as bone and soft tissue sarcomas which express FAPα, may also be treated with these compounds.

Consequently, another aspect of the present invention are pharmaceutical compositions comprising a compound of the present invention and optionally one or more suitable and pharmaceutically acceptable excipients, as exemplified in: *Remington: the science and practice of pharmacy. 19th ed. Easton : Mack Publ*., *1995.* The pharmaceutical compositions may be formulated as solids or solutions. Solid formulations may be for preparation of a solution before injection. Preferably, the pharmaceutical compositions of the invention are solutions for injection. They may be administered systemically, e.g. by intravenous injection, or topically, e.g. by direct injection into the tumor site. The dosage will be adjusted according to factors like body weight and health status of the patient, nature of the underlying disease, therapeutic window of the compound to be applied, solubility, and the like. It is within the knowledge of the expert to adjust dosage appropriately. For doxorubicin conjugates, for example, the dose will preferably be in the range from 10 mg/m² to 2000 mg/m², but also higher or lower doses may be appropriate.

Accordingly, a further aspect of the present invention is the use of a compound of the invention in the preparation of a pharmaceutical composition for the treatment of cancer. Furthermore, an aspect of the invention is a method of treatment of cancer, comprising administering an effective amount of a pharmaceutical composition of the invention to a patient. Indications include the treatment of cancer, specifically:
1) The treatment of epithelial carcinomas including breast, lung, colorectal, head and neck, pancreatic, ovarian, bladder, gastric, skin, endometrial, ovarian, testicular, esophageal, prostatic and renal origin;
2) Bone and soft-tissue sarcomas: Osteosarcoma, chondrosarcoma, fibrosarcoma, malignant fibrous histiocytoma (MFH), leiomyosarcoma;
3) Hematopoietic malignancies: Hodgkin's and non-Hodgkin's lymphomas;
4) Neuroectodermal tumors: Peripheral nerve tumors, astrocytomas, melanomas;
5) Mesotheliomas.

Also included are the treatment of chronic inflammatory conditions such as rheumatoid arthritis, osteoarthritis, liver cirrhosis, lung fibrosis, arteriosclerosis, and abnormal wound healing.

A further aspect of the invention is a method of treatment of cancer, wherein a prodrug is administered to a patient wherein said prodrug is capable of being converted into a cytotoxic or cytostatic drug by an enzymatic activity, said enzymatic activity being the expression product of cells associated with tumor tissue. Preferably, said enzymatic activity is the proteolytic activity of FAPα.

One method of administration of the compounds is intravenous infusion. Other possible routes of administration include intraperitoneal (either as a bolus or infusion), intramuscular or intratumoral injection. Where appropriate, direct application may also be possible (for example, lung fibrosis).

One skilled in the art will appreciate that although specific reagents and reaction conditions are outlined in the following examples, modifications can be made which are meant to be encompassed by the scope of the invention. The following examples, therefore, are intended to further illustrate the invention and are not limiting.

### Example I

### Synthesis of (4-Carboxymethyl-phenyl)-acetic acid N-hydroxysuccinimidyl ester

1,4-phenylendiacetic acid (4.8 g, 25 mmol) was dissolved in acetonitrile (100 ml) and *N,N*-dimethylformamide (100 ml) The solution was cooled to 0 °C and *N*-hydroxysuccinimide (2,9 g, 25 mmol) and diisopropylethylamine (8.6 ml, 50 mmol) were added. The solution was stirred for 30 min at 0 °C. Then a solution of dicyclohexyl carbodiimide(5.2 g, 25 mmol) dissolved in acetonitrile (50 ml) was added dropwise over 1 h. The ice bath was removed an the suspension was stirred over night. The suspension was filtered and the solvent of the filtrate was concentrated under reduced pressure. A residual amount of dicyclohexyl urea was precipitated by addition of diethyl ether and removed by filtration. The filtrate was purified by reversed phase HPLC applying acetonitrile/water gradient. The product fraction was lyophilized to give the product as white cristalls (1.1 g, 16 %). The product gave satisfactory analytical data. HPLC > 95 %; ES-MS: [M+H]⁺ = 292

(3-Carboxymethyl-phenyl)-acetic acid *N*-hydroxysuccinimidyl ester was prepared analogously.

### Example 2

### [2-(4-Carboxymethyl-phenyl)-acetyl]-Pro-Ala-Gly-Pro-Dox (III-G)

All steps were performed under nitrogen atmosphere.

### 2A Aloc-Pro-Ala-Gly-Pro-Dox:

Aloc-Pro-Ala-Gly-Pro-OH (190 mg 0.45 mmol) and Doxorubicin hydrochloride (260 mg 0.49 mmol) were dissolved in *N*,*N*-dimethylformamide (5 ml). Diisopropylethylamine (153 µl 0.90 mmol) was added and the solution was cooled to 0 °C. To the stirred mixture a solution of O-(7-azabenzotriazol- 1-yl)-1,1,3,3-tetramethyluronium hexfluorophosphate (HATU) (221mg 0.582 mmol) in *N*,*N*-dimethylformamide (10 ml) was added dropwise over a periode of 30 min. The solution was stirred for 1 h at 0 °C. After addition of diisopropylethylamine (25 µl, 0.14 mmol) the solvent was removed *in vacuo* at 30 °C. The crude product was dissolved in methanol (1 ml) and water (6 ml) and purified by reversed phase HPLC applying acetonitrile/water gradient. The product fraction was lyophilized and gave red cristalls of Aloc-Pro-Ala-Gly-Pro-Dox (345 mg, 81 %). The product gave satisfactory analytical data. HPLC > 95 %; ES-MS: [M+Na]⁺ = 972

### 2B H-Pro-Ala-Gly-Pro-Dox:

Aloc-Pro-Ala-Gly-Pro-Dox (343 mg 0.36 mmol) was dissolved dichloromethane (20 ml) and diethylamine (185 µl, 1.81 mmol) and Pd(Ph₃P)₄ (20.8 mg 0.02 mmol) were added. The solution was stirred at room temperature for 1 h. The solvent was removed under reduced pressure and the crude oil was dissolved water (3 ml) and acetonitrile (1 ml). The product was purified by reversed phase HPLC applying acetonitrile/water gradient and the product fraction was lyophilized to give red cristalls of H-Pro-Ala-Gly-Pro-Dox (296 mg, 95 %). The product gave satisfactory analytical data. HPLC > 95 %; ES-MS: [m+H]⁺= 866

### 2C [2-(4-Carboxymethyl-phenyl)-acetyl]-Pro-Ala-Gly-Pro-Dox (III-G):

H-Pro-Ala-Gly-Pro-Dox (100 mg 0.12 mmol) was dissolved in in *N*,*N*-dimethylformamide (5 ml). (4-Carboxymethyl-phenyl)-acetic acid *N*-hydroxysuccinimidyl ester (36.8 mg 0.127 mmol) and diisopropylethylamine (22 µl 0.127 mmol) were added. The solution was stirred for 48 h at room temperature. The solvent was removed under reduced pressure at 30 °C. The crude product was purified by reversed phase HPLC applying acetonitrile/water gradient and the product fraction was concentrated by lyophilization to yield the product (77 mg, 64 %). The product gave satisfactory analytical data. HPLC > 95 %; ES-MS: [M+H]⁺ = 1043

In analogy to this method the following compounds were prepared:
[2-(4-Carboxymethyl-phenyl)-acetyl]-N-MeIle-Ala-Gly-Pro-Dox (III-E)
[2-(4-Carboxymethyl-phenyl)-acetyl]-N-MeLeu-Ala-Gly-Pro-Dox (III-A)
[2-(4-Carboxymethyl-phenyl)-acetyl]-N-MeVal-Ala-Gly-Pro-Dox (III-F)
[2-(4-Carboxymethyl-phenyl)-acetyl]-Aze-Ala-Gly-Pro-Dox (III-H)
[2-(3-Carboxymethyl-phenyl)-acetyl]-Pro-Ala-Gly-Pro-Dox (III-K)
[2-(3-Carboxymethyl-phenyl)-acetyl]-N-MeIle-Ala-Gly-Pro-Dox (III-B)
[2-(3-Carboxymethyl-phenyl)-acetyl]-N-MeLeu-Ala-Gly-Pro-Dox (III-C)
[2-(3-Carboxymethyl-phenyl)-acetyl]-N-MeVal-Ala-Gly-Pro-Dox (III-D)

### Assay for cleavage of MNA substrates by FAP:

### Buffer A:

### 100mM Tris HCl pH 7.8, 100 mM NaCl

Cell extract from 293 cells stably transfected with FAP prepared as described (see Park, et al., Fibroblast Activation Protein, a Dual Specificity Serine Protease expressed in human tumor stromal fibroblasts. (1999) J. Biol. Chem. 36505-12.). A similar extract was also prepared from parental 293 control cells without FAP. The FAP concentration in the FAP-transfected cell extract was estimated by immunoassay and 1 ng enzyme (diluted in buffer A) was used per assay. FAP-negative 293 control cell extract was used at the same dilution (also in buffer A) as a negative control. Substrate was initially dissolved in dimethylformamide at a concentration of 200 mM and diluted in buffer A to a final concentration of 2.5 mM. A few substrates were not soluble at this concentration and had to be diluted further.

### Assay conditions:

10 µl 10% DMSO in buffer A
70 µl diluted FAP cell extract containing 1 ng FAP enzyme (OR control 293 cell extract without FAP)
20 µl 2.5 mM substrate

Mix, incubate at room temperature for 1 hour, and measure fluorescence in Fluorostar fluorimeter at the following wavelengths:
MNA conjugates: Excitation: 355 nm, Emission: 405 nm.

The fluorescence measured in the samples treated with control 293 control cell extracts without FAP is subtracted from the values measured in the samples treated with 1 ng FAP enzyme.

### Example 3

### Synthesis of [2-(4-Carboxymethyl-phenyl)-acetyl]-Pro-Ala-Gly-Pro-MNA

### 3A Boc-Pro-Ala-Gly-OH:

H-Gly-2-chlorotrityl-resin (10 g, 11.1 mmol) were added to a reaction vessel and washed with *N,N*-dimethylformamide (DMF) (three times with 150 ml). Fmoc-Ala-OH (20.7 g, 66.6 mmol), O-(benzotriazol-1-yl)-*N*,*N*,*N*,*N*-tetramethyluronium tetrafluoroborate (TBTU) (21.4 g, 66.6 mmol), 1-hydroxybenzotriazole (HOBt) (9.06 g, 66.6 mmol) and diisopropylethylamine (8 eq.) dissolved in DMF (100 ml) were added. The suspension was shaken at room temperature over night. The resin was washed carefully four times with DMF, dichloromethane, and DMF. The cleavage of the Fmoc-group was performed with a solution of 30% piperidine in DMF for 30 minutes followed by careful washing steps with DMF (4 times), dichloromethane (3 times) and DMF (4 times). Boc-Pro-OH was incorporated in the same manner. After cleavage of the Fmoc group as described above the resin was finally washed with methanol and diethyl ether and dried in a stream of nitrogen. The cleavage of the product was performed with a solution of 30 % 1,1,1,3,3,3-hexafluoro-2-propanol in dichloromethane for 30 minutes at room temperature. The filtrate was concentrated in vacuo to yield the peptide which was pure enough for further reaction. The product gave satisfactory analytical data. HPLC > 95 %; ES-MS: [M+H]⁺ = 344.2

### 3B H-Pro-Ala-Gly-Pro-MNA:

Boc-Pro-Ala-Gly-OH (3.3 g, 9.6 mmol), H-Pro-MNA (3.2 g, 10.6 mmol), TBTU (3.1 g, 9.6 mmol), HOBt (1.3 g, 9.6 mmol) and diisopropylethylamine (3 eq.) were dissolved in DMF (20 ml) and stirred at room temperature for 4 hours. The solvent was removed under vacuum and the residual oil was dissolved with trifluoroacetic acid/water 95:5 (10 ml) for 3 h. The solvent was removed under vacuum and the crude product was purified by reversed phase HPLC applying acetonitrile/water gradient. The product gave satisfactory analytical data.
HPLC > 95 %; ES-MS: [M+H]⁺= 496.6

### 3C 4-Carboxymethylphenylacetyl-Pro-Ala-Gly-Pro-MNA:

A solution of 1,4-phenylendiacetic acid (16 mg, 80 µmol), H-Pro-Ala-Gly-Pro-MNA (40 mg, 80 µmol), hydroxybenzotriazol (11 mg, 80 µmol), O-(Benzotriazol-1-yl)-*N*,*N*,*N*,*N*-tetramethyluronium tetrafluoroborate (TBTU) (26 mg, 80 µmol) and DIPEA (30 µl, 160 µmol) in *N*,*N*-dimethylformamide (4 ml) was shaken overnight at room temperature. The solution was concentrated to dryness under reduced pressure and the residue was purified via reversed phase HPLC applying acetonitrile/water gradient. The product containing fractions were lyophylized to yield 26 mg (39 µmol, 48 %) of the desired product as a white powder. The product gave satisfactory analytical data. HPLC > 95 %; ES-MS: [M+H]⁺= 672.7

The following table shows the peptide-MNA-conjugates which have been prepared analogously and includes cleavage data by FAP.

| **MNA-Conjugate** | **Cleavage [µM]** |
|---|---|
| [2-(4-Carboxymethyl-phenyl)-acetyl]-N-MeIle-Ala-Gly-Pro-MNA | 16.36 |
| [2-(4-Carboxymethyl-phenyl)-acetyl]-N-MeLeu-Ala-Gly-Pro-MNA | 13.97 |
| [2-(4-Carboxymethyl-phenyl)-acetyl]-N-MeVal-Ala-Gly-Pro-MNA | 13.39 |
| [2-(4-Carboxymethyl-phenyl)-acetyl]-Aze-Ala-Gly-Pro-MNA | 9.54 |
| [2-(3-Carboxymethyl-phenyl)-acetyl]-Pro-Ala-Gly-Pro-MNA | 2.80 |
| [2-(3-Carboxymethyl-phenyl)-acetyl]-N-MeIle-Ala-Gly-Pro-MNA | 15.36 |
| [2-(3-Carboxymethyl-phenyl)-acetyl]-N-MeLeu-Ala-Gly-Pro-MNA | 12.00 |
| [2-(3-Carboxymethyl-phenyl)-acetyl]-N-MeVal-Ala-Gly-Pro-MNA | 9.52 |
| [2-(4-Carboxymethyl-phenyl)-acetyl]-Pro-Ala-Gly-Pro-MNA | 7.25 |
| [2-(2-Carboxymethyl-phenyl)-acetyl]-Pro-Ala-Gly-Pro-MNA | 0.94 |

### Solubility Test

The solubilities of the compounds of formula

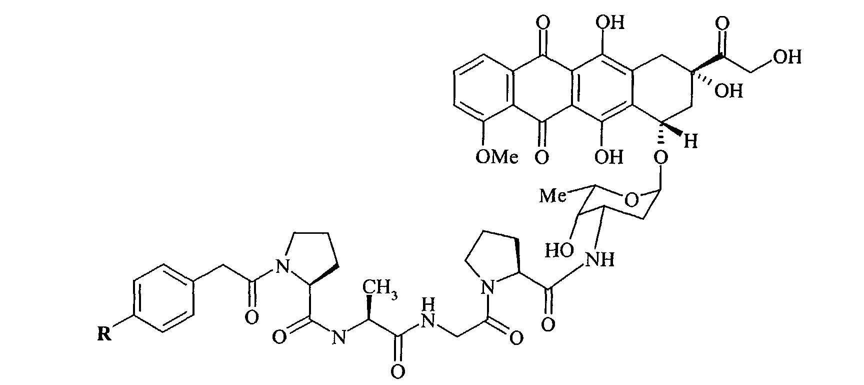

| Compound | **R** | Origin |
|---|---|---|
| **A** | -CH₂-COOH | Present invention, compound IIIH |
| **B** | H | WO 00/71571, compound IIIB |

in a phosphate buffer have been compared.

It has been found that the solubility of **A** is > 10 mg/ml, whereas the solubility of **B** is < 2 mg/ml.

## Claims

1. A compound of formula (I) 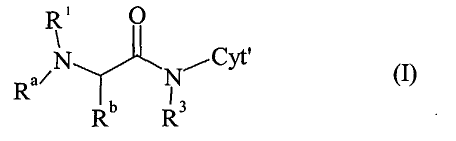 or a pharmaceutically acceptable salt thereof,
wherein
R¹ represents an amino alkanoyl or oligopeptidoyl group, the N-terminal amino function of which is attached to a capping group (Cg) of formula (II) 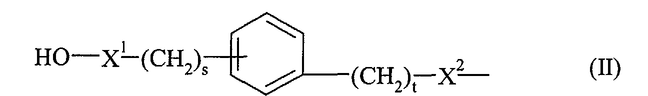 in which
X¹ represents C=O or SO₂,
X² represents C=O, SO₂, NH-C=O or a single bond,
s is an integer of 1 or 2, and
t is 0 or an integer of 1, 2 or 3,
R^{a} and R^{b} together with the interjacent N-C group form an optionally substituted, optionally benzo- or cyclohexano-condensed 3- to 7-membered saturated or unsaturated heterocyclic ring, in which one or two CH₂ groups may also be replaced by NH, O or S; R³ represents H, C₁-C₆-alkyl, C₃-C₈-cycloalkyl, aryl or heteroaryl; and
Cyt' represents the residue of a cytotoxic or cytostatic compound.

2. A compound of formula (I) according to claim 1, wherein
X¹ represents C=O or SO₂,
X² represents C=O or SO₂,
s is an integer of 1 or 2, and
t is an integer of 1 or 2.

3. A compound of formula (I) according to claim 1 or 2, wherein
X¹ and X² represent C=O,
s and t are 1.

4. A compound of formula (I) according to any of claims 1 to 3, wherein
R¹ represents a residue of formula Cg-A, Cg-B-A or Cg-(D)ₙ-B-A, in which Cg represents a capping group of formula (II),
A, B and D each independently represent moieties derived from amino carboxylic acids of the formula -[NR⁴-(X)ₚ-CO]- wherein X represents CR⁵R⁶ and wherein R⁴, R⁵ and R⁶ each independently represent a hydrogen atom, an optionally substituted C₁-C₆-alkyl, C₃-C₈-cycloalkyl, aryl, aralkyl, heteroaryl or heteroarylalkyl group, and
p is 1, 2, 3, 4, 5; or
A, B and D each independently represent moieties derived from cyclic amino carboxylic acids of formula 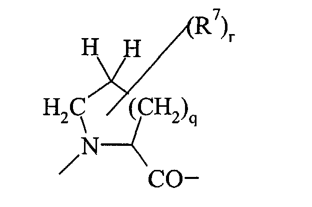 wherein
R⁷ represents C₁-C₆-alkyl, OH, or NH₂,
n is an integer from 1 to 10;
q is 0, 1 or 2; and
r is 0, 1 or 2.

5. A compound of formula I according to any of claims 1 to 4, wherein the heterocyclic ring formed by Ra, Rb and the interjacent N-C is substituted by R8 and R9, wherein R8 and R9 each independently represent a hydrogen or halogen atom or a C1-C6-alkyl, C1-C6-alkylamino, di-C1-C6-alkylamino, C1-C6-alkoxy, thiol, C1-C6-alkylthio, oxo, imino, fomyl, C1-C6-alkoxy carbonyl, amino carbonyl, C3-C8-cycloalkyl, aryl, or heteroaryl group.

6. A compound of formula IA 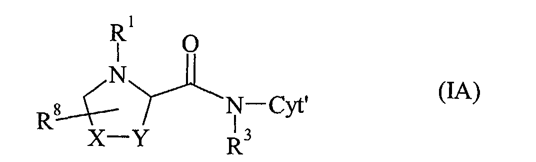 wherein R¹, R³, R⁸, Cyt' are as defined in any of the preceding claims, and X-Y represents CHR⁹-CH₂, CR²=CH, NH-CH₂, CH₂-NH, -CR⁹-, CH₂-CHR⁹-CH₂.

7. A compound of formula IA1 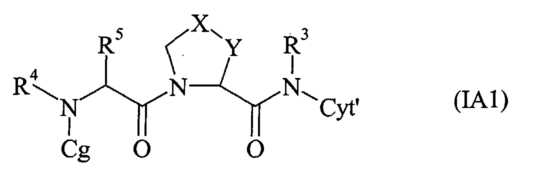 wherein R³, R⁴, R₅, Cyt', Cg, X and Y are as defined in any of the preceding claims, or R⁴ and R⁵ together with the interjacent N-C group form an optionally substituted, optionally benzo- or cyclohexano-condensed 3- to 7-membered saturated or unsaturated heterocyclic ring, in which one or two CH₂ groups may also be replaced by NH, O or S.

8. A compound of formula IA2 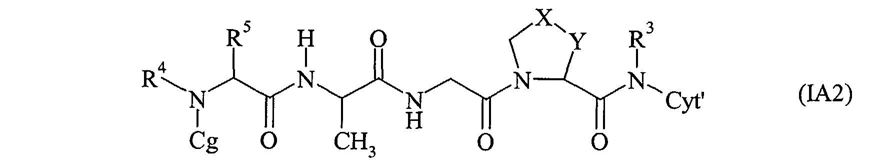 wherein R³, R⁴, R⁵, Cyt', Cg, X and Y each independently are as defined in any of the preceding claims, or
R⁴ and R⁵ together with the interjacent N-C group form an optionally substituted, optionally benzo- or cyclohexano-condensed 3- to 7-membered saturated or unsaturated heterocyclic ring.

9. A compound according to any of the preceding claim, wherein
R¹ represents a group selected from Cg-A, Cg-B-A- and Cg-(D)ₙ-B-A- in which A, B and D are amino acid moieties, which are each independently selected from glycine (Gly), and the D- or L-forms of alanine (Ala), valine (Val), leucine (Leu), isoleucine (Ile), phenylalanine (Phe), tyrosine (Tyr), tryptophan (Trp), cysteine (Cys), methionine (Met), serine (Ser), threonine (Thr), lysine (Lys), arginine (Arg), histidine (His), aspartatic acid (Asp), glutamic acid (Glu), asparagine (Asn), glutamine (Gln), proline (Pro), 4-hydroxy-proline (Hyp), 5-hydroxy-lysine, norleucine (Nle), 5-hydroxynorleucine (Hyn), 6-hydroxynorleucine, ornithine, cyclohexylglycine (Chg), N-Methylglycin (N-MeGly), N-Methylalanin (N-MeAla), N-Methylvaline (N-MeVal), N-Methylleucine (N-MeLeu), N-Methylisoleucine (N-MeIle), N-Methylnorleucin (N-MeNle), N-Methyl-2-aminobutyric acid (N-MeAbu) and N-Methyl-2-aminopentanoic acid (N-MeNva).

10. A compound of formula I according to any of the preceding claims, wherein the unit A is selected from L-proline, glycine, L-norleucine, L-cyclohexylglycine, L-5-hydroxynorleucine, L-6-hydroxynorleucine, L-5-hydroxylysine, L-arginine, and L-lysine.

11. A compound of formula I according to any of the preceding claims, wherein the unit D is selected from L-proline, (Pro), N-Methylglycin (N-MeGly), N-Methylalanin (N-MeAla), N-Methylvaline (N-MeVal), N-Methylleucine (N-MeLeu), N-Methylisoleucine (N-MeIle), N-Methylnorleucin (N-MeNle), N-Methyl-2-aminobutyric acid (N-MeAbu) and N-Methyl-2-aminopentanoic acid (N-MeNva).

12. A compound according to any of the preceding claims wherein R1 is a group selected from the formulae (1) to (14):
Cg-Gly (1)
Cg-Nle (2)
Cg-Val (3)
Cg-Met (4)
Cg-Xxx-Gly (5)
Cg-Xxx-Hyn (6)
Cg-Xxx-Pro (7)
Cg-Xxx-His (8)
Cg-Xxx-Met (9)
Cg-Xxx-Ala (10)
Cg-Xxx-Hyn (11)
Cg-Xxx-Ala-Gly (12)
Cg-(Xxx)ₙ-Xxx-Gly (13)
Cg-(Xxx)ₙ-Xxx-Ala-Gly (14)
wherein
Cg represents a capping group of formula II;
Xxx represents a moiety derived from an amino carboxylic acid; and
n is an integer from 1 to 6.

13. A compound according to claim 12 wherein the amino alkanoic acid moieties exist in the (L)-configuration

14. A compound of any one of claims 1 to 13, wherein Cyt' is an anthracycline group.

15. A compound of claim 14 selected from the formulae (III-A) to (III-K):

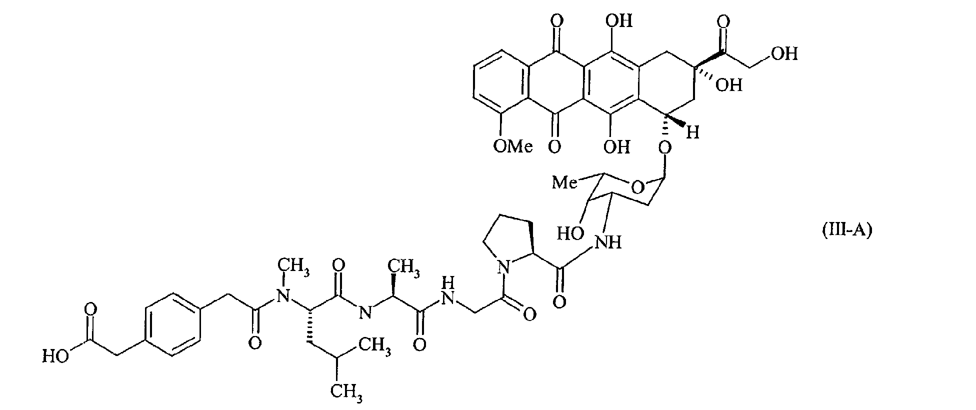

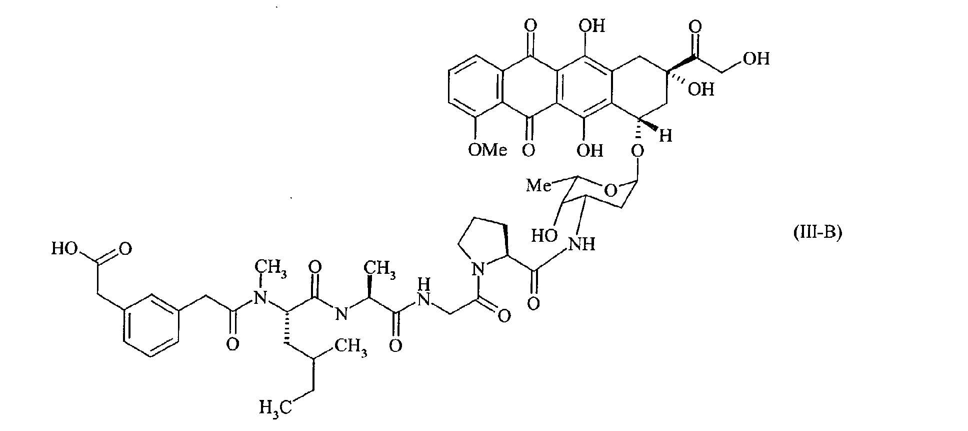

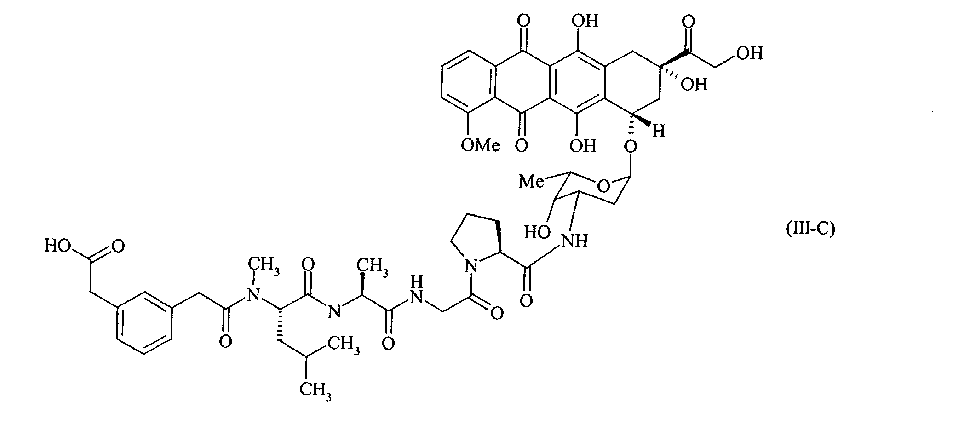

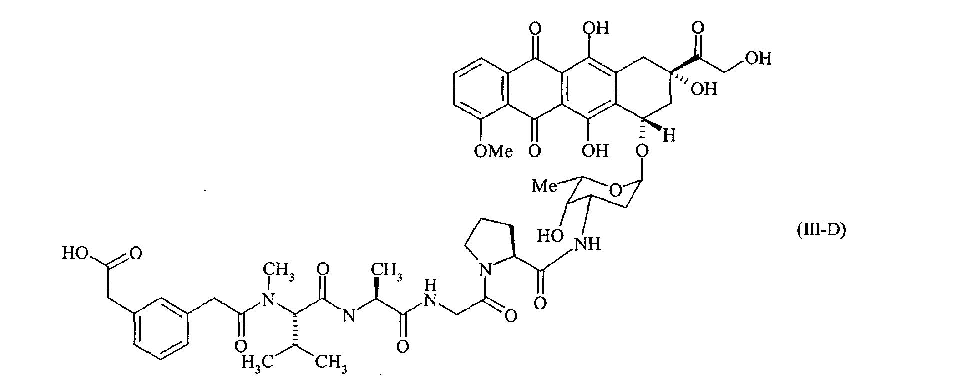

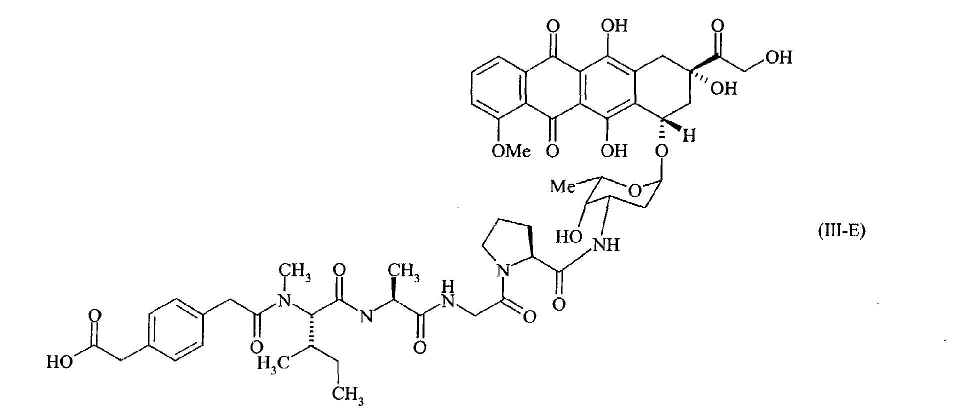

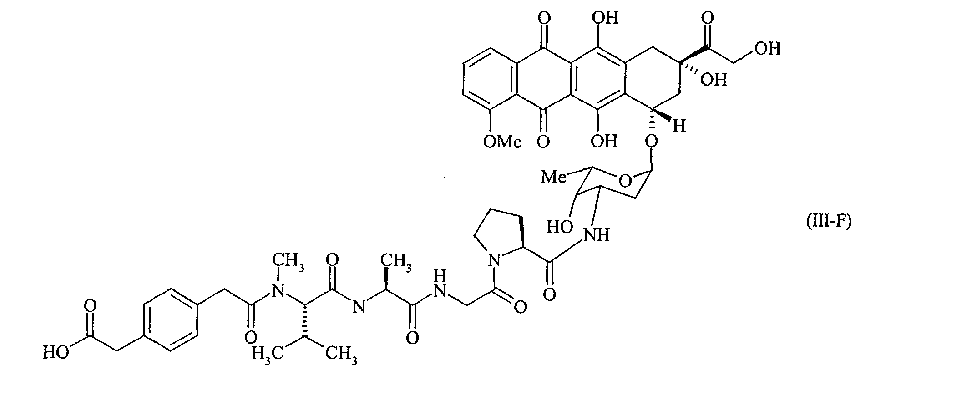

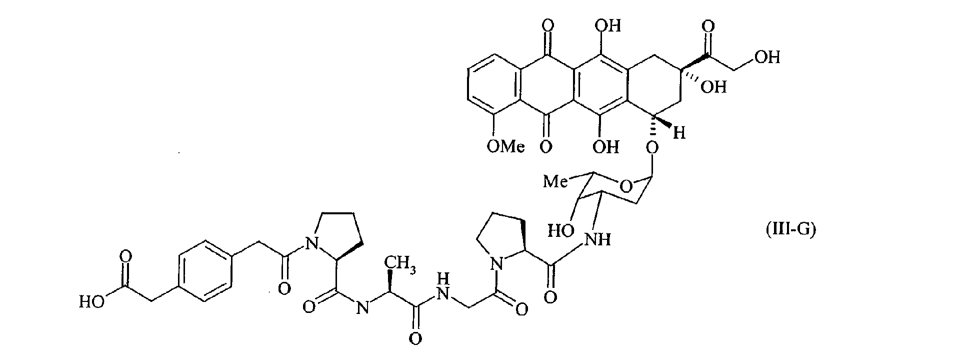

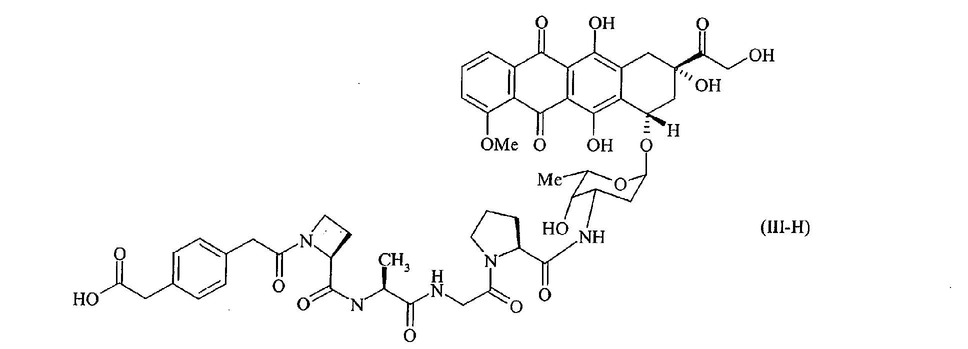

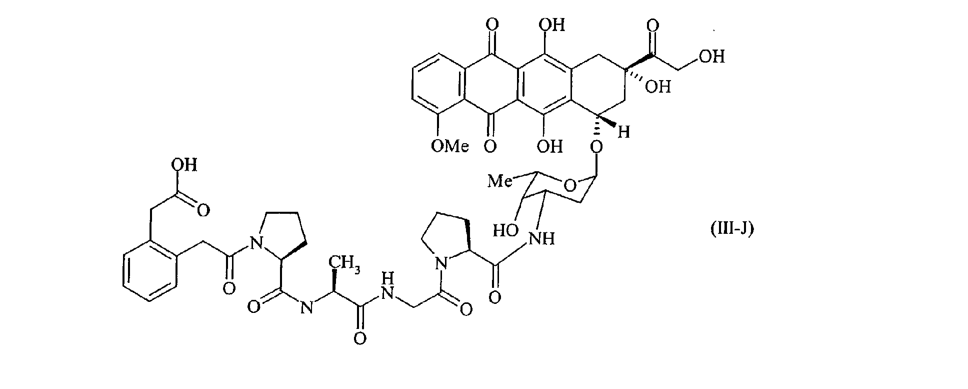

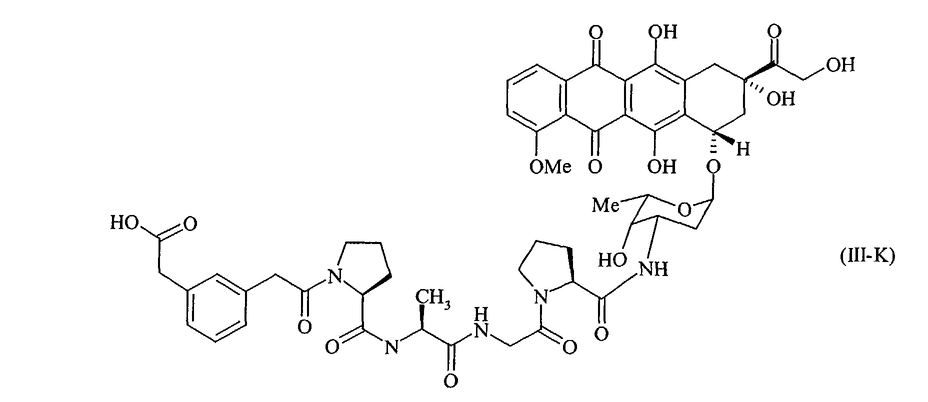

16. A prodrug that is capable of being converted into a cytotoxic or cytostatic drug, by the catalytic action of FAPα, said prodrug exhibits an oligomeric part comprising up to 13 amino carboxylic residues, the C-terminal amino carboxylic thereof is recognised by FAPα, and a cytotoxic or cytostatic part, **characterized in that**
the N-terminal amino function of the oligomeric part is attached to a capping group (Cg) of formula (II) 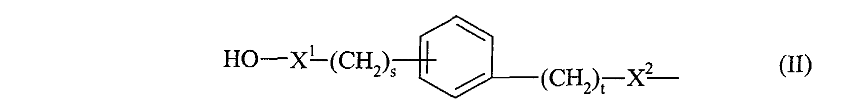 in which
X¹ represents C=O or SO₂,
X² represents C=O, SO₂, NH-C=O or a single bond,
s is an integer of 1 or 2, and
t is 0 or an integer of 1, 2 or 3.

17. The prodrug of claim 16, wherein the C-terminal amino carboxilic residue is selected from D-proline, L-proline, D-hydroxyproline and L-hydroxyproline and the oligomeric part comprises two, three, or four amino carboxylic acid residues.

18. A compound of any one of the preceding claims for medical use.

19. Pharmaceutical composition comprising a compound according to any one of claims 1 to 18, and optionally one or more pharmaceutically acceptable excipients.

20. Use of a compound according to any one of claims 1 to 18 in the preparation of a pharmaceutical composition for the treatment of cancer.

21. Method of treatment of cancer, comprising administering a pharmaceutical composition according to claim 19 to a patient.

22. Method of treatment of cancer, wherein a prodrug according to claim 16 or 17 is administered to a patient.

23. Use of a prodrug according to claim 16 or 17 for the manufacture of a stable medicament for the treatment of cancer.
